# EUROPÄISCHE PATENTSCHRIFT

(11) **EP 0 716 049 B1**
(45) Veröffentlichungstag und Bekanntmachung des Hinweises auf die Patenterteilung: **03.09.2003**
(21) Anmeldenummer: 95118222.9
(22) Anmeldetag: 20.11.1995
(51) Int. Cl.: C03C 3/097, C03C 4/08, A61K 6/083, A61K 6/06

(54) **Bariumfreies Dentalglas mit guter Röntgenabsorption**
Tooth filling made of barium-free glass with good X-ray absorption
Verre dentaire sans barium à bonne absorption de rayons X

(30) Priorität: 05.12.1994 DE 4443173
(43) Veröffentlichungstag der Anmeldung: 12.06.1996
(73) Patentinhaber: Schott Glas, 55122 Mainz (DE); CARL-ZEISS-STIFTUNG trading as Schott Glas, 55122 Mainz (DE)
(72) Erfinder: Grabowski, Danuta, D-65232 Taunusstein (DE); Clement, Marc, Dr., D-55118 Mainz (DE); Daimer, Johann, D-84051 Oberahrain (DE); Paschke, Hartmut, Dr., D-84030 Ergolding (DE)

(56) Entgegenhaltungen:
- EP-A- 0 622 342
- WO-A-94/18134
- DE-C- 4 323 143
- DATABASE WPI Section Ch, Week 9030 Derwent Publications Ltd., London, GB; AN 90-226842 XP002000305 & JP-A-02 153 839 (NIPPON ELECTRIC GLASS) , 13.Juni 1990
- PATENT ABSTRACTS OF JAPAN vol. 016, no. 581 (C-1012), 21.Dezember 1992 & JP-A-04 231060 (NIPPON ELECTRIC GLASS CO LTD), 19.August 1992,
- PATENT ABSTRACTS OF JAPAN vol. 017, no. 627 (C-1131), 19.November 1993 & JP-A-05 194130 (HOYA CORP), 3.August 1993,
- PATENT ABSTRACTS OF JAPAN vol. 017, no. 627 (C-1131), 19.November 1993 & JP-A-05 194132 (MORITA MFG CO LTD), 3.August 1993,

## Beschreibung

Für Zahnfüllungen werden in zunehmendem Maße Dental-Komposite eingesetzt, um mögliche Nebenwirkungen von Amalgam-Füllungen zu umgehen und um einen besseren ästhetischen Eindruck zu erzielen. Dental-Komposite bestehen in der Regel aus einem anorganischen Anteil und einem organischen Kunstharz-Binder. Der anorganische Anteil besteht überwiegend aus Glaspulver. An das verwendete Glaspulver werden neben den für eine gute Füllung notwendigen Pulvereigenschaften auch noch bestimmte Anforderungen an die physikalischen und chemischen Eigenschaften des für das Pulver zu verwendenden Glases gestellt.

Das Glaspulver muß zunächst eine hohe Festigkeit besitzen. Weiterhin muß der Brechungsindex der Füllung an den des Kunstharzes angepaßt sein, um sicherzustellen, daß die Zahnfüllung den ästhetischen Anforderungen entspricht, d. h. daß sie vom Zahnschmelz kaum noch zu unterscheiden ist. Weiterhin wichtig ist, daß die thermische Ausdehnung des Glases im Verwendungsbereich der Füllung, d. h. bei Temperaturen zwischen 30 °C und 70 °C der des Zahnmaterials angepaßt ist, um sicherzustellen, daß die Füllung eine ausreichende Temperaturwechselbeständigkeit aufweist. Gerade durch den Wechsel von kalten und heißen Speisen ist hier die Gefahr gegeben, daß die Füllung durch derartige thermische Belastungen an Haltbarkeit verliert. Üblich ist ein möglichst kleiner Ausdehnungskoeffizient für das Glas, weil damit die verhältnismäßig hohe thermische Ausdehnung des Kunstharz-Binders kompensiert werden kann.

Ferner soll sich die Zahnfüllung im Röntgenbild deutlich von dem des Zahnmaterials abheben, um Randspalte und Sekundärkaries erkennen zu können. Das bedeutet, daß das Glas eine Mindest-Röntgenopazität aufweisen muß. Gemäß ISO 4049: 1988 (E) muß die Röntgenopazität einer Füllung bei einer Schichtdicke von 2 mm größer sein als die einer Aluminiumplatte gleicher Dicke. Die Röntgenopazität der Füllung wird als sogenannte Aluminiumgleichwertdicke angegeben. Unter Aluminiumgleichwertdicke wird die Dicke einer Aluminiumplatte verstanden, die die gleiche Röntgenabsorption wie eine 2 mm dicke Platte aus dem Füllungsmaterial hervorruft. Eine Aluminiumgleichwertdicke von 3 bedeutet demnach, daß eine 2 mm dicke Füllung die gleiche Absorption hervorruft wie eine 3 mm dicke Aluminiumplatte.

Weiterhin muß eine gute chemische Beständigkeit des Glaspulvers gegen Wasser, Säuren und Laugen zu einer langen Lebensdauer der Zahnfüllung beitragen. Wegen möglicher toxischer Nebenwirkungen soll auf die Verwendung von Barium-Bestandteilen in dem Glas verzichtet werden, obwohl diese Bestandteile eine gute Röntgenopazität hervorrufen. Die Verwendung von bleihaltigen Bestandteilen ist aus toxischen Gesichtspunkten grundsätzlich verboten.

Aus US-PS 47 75 592 ist ein Fluoro-Aluminosilikat-Glaspulver zur Verwendung im Dentalbereich bekannt. Die Grundgläser für diese Pulver besitzen jedoch nur eine sehr geringe Kristallisationsstabilität, so daß sich ihre Herstellung aufwendig gestaltet. Die Gläser besitzen einen sehr hohen Fluorgehalt von etwa 10 bis 40 Gew.-% und müssen zur Erzielung einer ausreichenden Härte und chemischen Beständigkeit Al₂O₃-Anteile von bis zu 40 Gew.-% enthalten. Dennoch ist die hydrolytische Beständigkeit der Glaspulvernicht befriedigend.

Weiterhin ist aus der älteren Deutschen Patentanmeldung P 43 23 143.7 ein bariumfreies Dentalglas mit hoher Röntgenabsorption bekannt, das eine Zusammensetzung in Gew.-% auf Oxidbasis besitzt von SiO₂ 45-65; B₂O₃ 5-20; Al₂O₃ 5-20; CaO 0-10; SrO 15-35 und F₂-O 0-2. Die gute Röntgenopazität wird hier durch einen verhältnismäßig hohen Anteil an SrO erreicht.

Die Aufgabe der Erfindung besteht darin, ein weiteres Dentalglas zu finden, das eine gute Röntgenabsorption besitzt, frei ist von Barium und Blei und das eine gute chemische und thermische Beständigkeit hat.

Diese Aufgabe wird durch das in Patentanspruch 1 beschriebene Dentalglas gelöst.

Das Glas kann aus einer minimalen Anzahl an Komponenten aufgebaut werden, was die toxikologische Beurteilung des Glases auf mögliche Nebenwirkungen erheblich erleichtert.

Als glasbildende Komponenten werden in dem Glas SiO₂ in Mengen von 55-70 Gew.-% und ZrO₂ in Mengen von 10-25 Gew.-% eingesetzt. Durch den Zirkongehalt werden die mechanischen Eigenschaften und hierbei besonders die Zug- und Druckfestigkeit deutlich verbessert sowie die Sprödigkeit des Glases herabgesetzt. Um das Aufschmelzen des SiO₂/ZrO₂-Glases zu erleichtern, können dem Glas bis zu insgesamt 25 Gew.-% Alkalioxide in Form von 10-25 Gew.-% Na₂O, 0-25 Gew.-% K₂O und/oder 0-5 Gew.-% Li₂O zugesetzt werden. Steigt der Anteil an Alkalioxiden über 25 Gew.-%, so verringert sich die chemische und mechanische Beständigkeit des Glases und der thermische Ausdehnungskoeffizient steigt deutlich an. Bevorzugt wird es, wenn der Gehalt an K₂O zwischen 0 und 15 und an Li₂O zwischen 0 und 5 liegt, wobei der Gesamtgehalt der Alkalioxide vorzugsweise zwischen 15 und 25 Gew.-% liegen soll. Mit diesem Alkaligehalt erreicht man eine gute Einschmelzbarkeit des Glases bei guter chemischer Beständigkeit.

Zur Korrektur des Brechungsindex kann die Zugabe von bis zu 3 Gew.-% Fluor von Vorteil sein. Das Glas kann ferner bis zu 10 Gew.-% CaO enthalten. CaO kann dem Glas zugesetzt werden, um die physikalischen Eigenschaften des Glases zu variieren. CaO trägt z.B. bei zur Erhöhung der Aluminiumgleichwertdicke. Im Vergleich zu Si besitzt Calcium den etwa 3-fachen Massenschwächungskoeffizienten.

Ein Überschreiten des Anteils an CaO führt allerdings zu einer Verschlechterung der Röntgenabsorption des Glases bei gleichzeitiger Steigerung der Entglasungsneigung.

Ohne die Eigenschaften des Glases wesentlich zu beeinträchtigen, können auch in dem Glas noch bis zu 10 Gew.-% SrO, bis zu 5 Gew.-% MgO, bis zu 10 Gew.-% Al₂O₃, bis zu 10 Gew.-% GeO₂, bis zu 10 Gew.-% P₂O₅, bis zu 5 Gew.-% TiO₂, und jeweils bis zu 10 Gew.-% La₂O₃, Y₂O₃, Ta₂O₃, Gd₂O₃, ZnO, B₂O₃, Nb₂O₅ und P₂O₅ vorhanden sein. Die Zahl der in dem Glas vorhandenen zusätzlichen Oxide sollte aber nach Möglichkeit nicht zu hoch gewählt werden, um das Ziel einer einfachen toxikologischen Beurteilung des Glases nicht aus den Augen zu verlieren.

Um die optischen Eigenschaften der Zahnfüllung dem Zahnschmelz möglichst anzupassen, wird der Brechwert des Glases dem des Kunstharzes angepaßt. Verwendete Kunstharze haben Brechungsindizes im Bereich von 1,5 bis 1,6. Das erfindungsgemäße Glas besitzt einen Brechwert von unter 1,6 und erfüllt somit diese Forderung.

Für die zahnärztliche Praxis ist die gute Erkennbarkeit der Füllung im Röntgenbild von hoher Bedeutung. Mit dem erfindungsgemäßen Glas hergestellte Füllungen besitzen Aluminiumgleichwertdicken von > 2,5 mm, im allgemeinen > 3 mm und besitzen damit die erforderlichen Eigenschaften für die Verwendung in der Zahnrestauration. Unter Aluminiumgleichwertdicke wird die Dicke einer Aluminiumplatte verstanden, die die gleiche Röntgenabsorption wie eine 2 mm dicke Füllung besitzt.

Nach seiner Herstellung wird aus dem Glas in an sich bekannter Weise z. B. durch Mahlen und ggf. Sieben ein Glaspulver hergestellt, das die für Dentalzwecke übliche durchschnittliche Teilchengröße von < 10 µm, insbesondere 0,5 bis 5 µm, bevorzugt 0,7 bis 1,5 µm besitzt. Die Pulverkörnung spielt eine wichtige Rolle, sie beeinflußt die Polierbarkeit der Komposite, sowie die Abrasions- und mechanische Festigkeit. Zur Erzielung guter mechanischer Eigenschaften ist in üblicher Weise eine nicht zu enge Korngrößenverteilung günstig, wie sie z. B. durch übliche Vermahlung und Absiebung der Grobanteile erreicht wird. Eine maximale Teilchengröße von 40 µm, vorzugsweise 20 µm, insbesondere 10 µm sollte nicht überschritten werden. In dieser Form ist das Glaspulver zur Verwendung als Füllmittel für als Zahnfüllungen verwendete Dental-Komposite besonders geeignet.

Es ist vielfach üblich, die zur Verwendung als Füllmittel für Dentalkomposite eingesetzten Glaspulver zu silanisieren, wobei die Silanisierung sowohl an sich als auch für diesen Verwendungszweck wohlbekannt ist. Die Silanisierung erleichtert das Erreichen eines hohen Füllgrades im Komposit und wirkt sich günstig auf die mechanischen Eigenschaften des Komposits aus.

Zur Herstellung von als Zahnfüllung verwendbaren Dental-Kompositen wird das Glaspulver mit in der Zahnmedizin üblichen, härtbaren Kunstharzen gemischt. Als Kunstharze werden überwiegend UV-härtbare Harze auf Acrylat-, Methacrylat-, 2,2-Bis-[4-(3-Methacryloxi-2-hydroxypropoxy)-phenyl]-propan-(Bis-GMA-), Urethan-Methacrylat-; Alcandiolmethacrylat- oder Cyanacrylatbasis verwendet. Das zur Füllung verwendete Glaspulver liegt in den fertigen Kunstharzpasten in Gewichtsanteilen von bis zu 80 Gew.-% vor, wobei angestrebt wird, den Glaspulveranteil aus Festigkeitsgründen so hoch wie möglich zu wählen.

### Beispiele:

Aus üblichen, reinen Rohstoffen wurden 6 Gläser erschmolzen, deren Zusammensetzung und Eigenschaften in der Tabelle zusammengefaßt sind. Gemessen wurde der Brechungsindex bei 587 nm Wellenlänge (n_{d}) und die Aluminiumgleichwertdicke (AG) nach ISO 4049.

**Tabelle:**

| Beispielgläser (Angaben in Gew.-%) | | | | | | |
|---|---|---|---|---|---|---|
| | 1 | 2 | 3 | 4 | 5 | 6 |
| SiO₂ | 65,7 | 64,0 | 55,0 | 63,0 | 70,0 | 65,0 |
| ZrO₂ | 10,0 | 16,0 | 20,0 | 12,0 | 5,0 | 25,0 |
| Na₂O | 24,3 | 20,0 | 25,0 | 15,0 | 25,0 | 10,0 |
| CaO | | | | 10,0 | | |
| n_{d} | 1,5300 | 1,5478 | 1,5681 | 1,5606 | 1,5133 | 1,5665 |
| AG (mm) | 2,8 | 3,8 | 4,6 | 3,3 | 2,5 | 4,9 |

## Patentansprüche

1. Bariumfreies Dentalglas mit guter Röntgenabsorption,
**gekennzeichnet durch**
eine Zusammensetzung (in Gew.-% auf Oxidbasis) von
| | |
|---|---|
| SiO₂ | 55 - 70 |
| ZrO₂ | 10 - 25 |
| Li₂O | 0 - 5 |
| Na₂O | 10 - 25 |
| K₂O | 0 - 25 |
| Σ Alkalioxide | 10 - 25 |

2. Dentalglas nach Anspruch 1,
**gekennzeichnet durch**
einen Gehalt (in Gew.-% auf Oxidbasis) von
| | |
|---|---|
| Σ Alkalioxide | 15 - 25 |

3. Dentalglas nach Anspruch 1 oder 2,
**gekennzeichnet durch**
einen zusätzlichen Gehalt (in Gew.-% auf Oxidbasis) von
| | |
|---|---|
| CaO | 0-10 |
| F₂ | 0 - 3 |

4. Dentalglas nach wenigstens einem der Ansprüche 1 bis 3,
**gekennzeichnet durch**
einen zusätzlichen Gehalt (in Gew.-% auf Oxidbasis) von
| | |
|---|---|
| SrO | 0 - 10 |
| MgO | 0 - 5 |
| Al₂O₃ | 0 - < 10 |
| GeO₂ | 0 - 10 |
| P₂O₅ | 0 - 10 |
| TiO₂ | 0 - 5 |
| La₂O₃ | 0 - 10 |
| Y₂O₃ | 0 - 10 |
| Ta₂O₃ | 0 - 10 |
| Gd₂O₃ | 0 - 10 |
| ZnO | 0 - < 10 |
| B₂O₃ | 0 - 10 |
| Nb₂O₅ | 0 - 10 |

5. Dentalglas nach wenigstens einem der Ansprüche 1 bis 4,
**gekennzeichnet durch**
einen Brechungsindex n_{d} von 1,5 bis 1,6 und eine Aluminiumgleichwertdicke von ≥ 2,5 mm.

6. Verwendung eines Dentalglases mit einer Zusammensetzung (in Gew.-% auf Oxidbasis) von
| | |
|---|---|
| SiO₂ | 55 - 70 |
| ZrO₂ | 10 - 25 |
| Li₂O | 0 - 5 |
| Na₂O | 10 - 25 |
| K₂O | 0 - 25 |
| Σ Alkalioxide | 10 - 25 |
als Glaspulver zur Herstellung von Kunstharzkompositen für die Zahnfüllung.

7. Verwendung eines Dentalglases nach Anspruch 6 mit einem Gehalt (in Gew.-% auf Oxidbasis) von
| | |
|---|---|
| Σ Alkalioxide | 15 - 25 |

8. Verwendung eines Dentalglases nach wenigstens einem der Ansprüche 6 bis 7,
**dadurch gekennzeichnet,**
**daß** das Glas einen zusätzlichen Gehalt (in Gew.-% auf Oxidbasis) von
| | |
|---|---|
| CaO | 0 - 10 |
| F₂ | 0 - 3 |
besitzt.

9. Verwendung eines Dentalglases nach wenigstens einem der Ansprüche 6 bis 8,
**dadurch gekennzeichnet,**
**daß** das Glas einen zusätzlichen Gehalt (in Gew.-% auf Oxidbasis) von
| | |
|---|---|
| SrO | 0 - 10 |
| MgO | 0 - 5 |
| Al₂O₃ | 0 - < 10 |
| GeO₂ | 0 - 10 |
| P₂O₅ | 0 - 10 |
| TiO₂ | 0 - 5 |
| La₂O₃ | 0 - 10 |
| Y₂O₃ | 0 - 10 |
| Ta₂O₃ | 0 - 10 |
| Gd₂O₃ | 0 - 10 |
| ZnO | 0 - < 10 |
| B₂O₃ | 0 - 10 |
| Nb₂O₅ | 0 - 10 |
besitzt.

10. Verwendung eines Dentalglases nach wenigstens einem der Ansprüche 6 bis 9,
**dadurch gekennzeichnet,**
**daß** das Glas einen Brechungsindex n_{d} von 1,5 bis 1,6 und eine Aluminiumgleichwertdicke von ≥ 2,5 mm besitzt.

11. Verwendung eines Glaspulvers nach wenigstens einem der Ansprüche 6 bis 10 mit einer mittleren Teilchengröße von ≦ 10 µm, insbesondere 0,5 bis 5 µm als Füllmaterial für zur Zahnfüllung verwendete Kunstharzpasten (Kunstharzkomposite).

## Claims

1. Barium-free dental glass with good x-ray absorption, **characterized by** a composition (in % by weight, based on oxide) of
| | |
|---|---|
| SiO₂ | 55 - 70 |
| ZrO₂ | 10 - 25 |
| Li₂O | 0 - 5 |
| Na₂O | 10 - 25 |
| K₂O | 0 - 25 |
| Σ alkali metal oxides | 10 - 25 |

2. Dental glass according to Claim 1, **characterized by** a content (in % by weight, based on oxide) of
| | |
|---|---|
| Σ alkali metal oxides | 15 - 25 |

3. Dental glass according to Claim 1 or 2, **characterized by** an additional content (in % by weight, based on oxide) of
| | |
|---|---|
| CaO | 0 - 10 |
| F₂ | 0 - 3 |

4. Dental glass according to at least one of Claims 1 to 3, **characterized by** an additional content (in % by weight, based on oxide) of
| | |
|---|---|
| SrO | 0 - 10 |
| MgO | 0 - 5 |
| Al₂O₃ | 0 - < 10 |
| GeO₂ | 0 - 10 |
| P₂O₅ | 0 - 10 |
| TiO₂ | 0 - 5 |
| La₂O₃ | 0 - 10 |
| Y₂O₃ | 0 - 10 |
| Ta₂O₃ | 0 - 10 |
| Gd₂O₃ | 0 - 10 |
| ZnO | 0 - < 10 |
| B₂O₃ | 0 - 10 |
| Nb₂O₅ | 0 - 10 |

5. Dental glass according to at least one of Claims 1 to 4, **characterized by** a refractive index n_{d} of 1.5 to 1.6 and an aluminium equivalent thickness of ≥ 2.5 mm.

6. Use of a dental glass having a composition (in % by weight, based on oxide) of
| | |
|---|---|
| SiO₂ | 55 - 70 |
| ZrO₂ | 10 - 25 |
| Li₂O | 0 - 5 |
| Na₂O | 10 - 25 |
| K₂ | 0 - 25 |
| Σ alkali metal oxides | 10 - 25 |
as glass powder for the production of synthetic resin composites for dental filling.

7. Use of a dental glass according to Claim 6 having a content (in % by weight, based on oxide) of
| | |
|---|---|
| Σ alkali metal oxides | 15 - 25 |

8. Use of a dental glass according to at least one of Claims 6 to 7, **characterized in that** the glass has an additional content (in % by weight, based on oxide) of
| | |
|---|---|
| CaO | 0 - 10 |
| F₂ | 0 - 3 |

9. Use of a dental glass according to at least one of Claims 6 to 8, **characterized in that** the glass has an additional content (in % by weight, based on oxide) of
| | |
|---|---|
| SrO | 0 - 10 |
| MgO | 0 - 5 |
| Al₂O₃ | 0 - < 10 |
| GeO₂ | 0 - 10 |
| P₂O₅ | 0 - 10 |
| TiO₂ | 0 - 5 |
| La₂O₃ | 0 - 10 |
| Y₂O₃ | 0 - 10 |
| Ta₂O₃ | 0 - 10 |
| Gd₂O₃ | 0 - 10 |
| ZnO | 0 - < 10 |
| B₂O₃ | 0 - 10 |
| Nb₂O₅ | 0 - 10 |

10. Use of a dental glass according to at least one of Claims 6 to 9, **characterized in that** the glass has a refractive index n_{d} of 1.5 to 1.6 and an aluminium equivalent thickness of ≥ 2.5 mm.

11. Use of a glass powder according to at least one of Claims 6 to 10, having a mean particle size of ≤ 10 µm, in particular 0.5 to 5 µm, as a filler material for synthetic-resin pastes (synthetic resin composites) used for dental filling.

## Revendications

1. Verre dentaire exempt de baryum présentant une bonne absorption de rayons X, **caractérisé par** une composition (en % en poids sur base d'oxydes) de
| | |
|---|---|
| SiO₂ | 55 à 70 |
| ZrO₂ | 10 à 25 |
| Li₂O | 0 à 5 |
| Na₂O | 10 à 25 |
| K₂O | 0 à 25 |
| Σ oxydes de métal alcalin | 10 à 25 |

2. Verre dentaire selon la revendication 1, **caractérisé par** une teneur (en % en poids sur base d'oxydes) de
| | |
|---|---|
| Σ oxydes de métal alcalin | 15 à 25 |

3. Verre dentaire selon la revendication 1 ou 2, **caractérisé par** une teneur supplémentaire (en % en poids sur base d'oxydes) de
| | |
|---|---|
| CaO | 0 à 10 |
| F₂ | 0 à 3 |

4. Verre dentaire selon au moins l'une quelconque des revendications 1 à 3, **caractérisé par** une teneur supplémentaire (en % en poids sur base d'oxydes) de
| | |
|---|---|
| SrO | 0 à 10 |
| MgO | 0 à 5 |
| Al₂O₃ | 0 à < 10 |
| GeO₂ | 0 à 10 |
| P₂O₅ | 0 à 10 |
| TiO₂ | 0 à 5 |
| La₂O₃ | 0 à 10 |
| Y₂O₃ | 0 à 10 |
| Ta₂O₃ | 0 à 10 |
| Gd₂O₃ | 0 à 10 |
| ZnO | 0 à < 10 |
| B₂O₃ | 0 à 10 |
| Nb₂O₅ | 0 à 10 |

5. Verre dentaire selon au moins l'une quelconque des revendications 1 à 4, **caractérisé par** un indice de réfraction n_{d} de 1,5 à 1,6 et une épaisseur équivalente d'aluminium ≥ 2,5 mm.

6. Utilisation d'un verre dentaire présentant une composition (en % en poids sur base d'oxydes) de
| | |
|---|---|
| SiO₂ | 55 à 70 |
| ZrO₂ | 10 à 25 |
| Li₂O | 0 à 5 |
| Na₂O | 10 à 25 |
| K₂O | 0 à 25 |
| Σ oxydes de métal alcalin | 10 à 25 |
comme poudre de verre pour la préparation d'une composition de résine synthétique pour le remplissage dentaire.

7. Utilisation d'un verre dentaire selon la revendication 6 présentant une teneur (en % en poids sur base d'oxydes) de
Σ oxydes de métal alcalin 15 à 25

8. Utilisation d'un verre dentaire selon au moins l'une quelconque des revendications 6 à 7, **caractérisée en ce que** le verre présente une teneur supplémentaire (en % en poids sur base d'oxydes) de
| | |
|---|---|
| CaO | 0 à 10 |
| F₂ | 0 à 3 |

9. Utilisation d'un verre dentaire selon au moins l'une quelconque des revendications 6 à 8, **caractérisée en ce que** le verre présente une teneur supplémentaire (en % en poids sur base d'oxydes) de
| | |
|---|---|
| SrO | 0 à 10 |
| MgO | 0 à 5 |
| Al₂O₃ | 0 à < 10 |
| GeO₂ | 0 à 10 |
| P₂O₅ | 0 à 10 |
| TiO₂ | 0 à 5 |
| La₂O₃ | 0 à 10 |
| Y₂O₃ | 0 à 10 |
| Ta₂O₃ | 0 à 10 |
| Gd₂O₃ | 0 à 10 |
| ZnO | 0 à < 10 |
| B₂O₃ | 0 à 10 |
| Nb₂O₅ | 0 à 10 |

10. Utilisation d'un verre dentaire selon au moins l'une quelconque des revendications 6 à 9,
**caractérisée en ce que** le verre présente un indice de réfraction n_{d} de 1,5 à 1,6 et une épaisseur équivalente d'aluminium ≥ 2,5 mm.

11. Utilisation d'une poudre de verre selon au moins l'une quelconque des revendications 6 à 10 présentant une grosseur moyenne des granules ≦ 10 µm, en particulier de 0,5 à 5 µm, comme matériau de remplissage pour des pâtes de résine synthétique utilisées pour le remplissage dentaire (résine synthétique composite).
